# EUROPEAN PATENT SPECIFICATION

(11) **EP 2 451 298 B1**
(45) Date of publication and mention of the grant of the patent: **12.06.2013**
(21) Application number: 10730463.6
(22) Date of filing: 09.07.2010
(51) Int. Cl.: A23L 1/30, A61K 35/74, B32B 7/02, B32B 27/08, A61P 1/12, A61P 3/12

(54) **PRODUCT FOR THE STORAGE OF FREEZE-DRIED LACTIC ACID BACTERIA MIXED WITH A POWDER FOR AN ORAL REHYDRATION SOLUTION**
PRODUKT FÜR DIE LAGERUNG VON GEFRIERGETROCKNETEN MILCHSÄUREBAKTERIEN, DIE MIT EINEM PULVER EINER ORALEN REHYDRIERUNGSLÖSUNG GEMISCHT SIND
PRODUIT POUR LE STOCKAGE DE BACTERIES LACTIQUES LYOPHILISEES MELANGEES A UNE POUDRE POUR SOLUTION DE REHYDRATATION ORALE

(30) Priority: 10.07.2009 US 270566 P; 06.07.2010 US 803758
(43) Date of publication of application: 16.05.2012
(73) Proprietor: Biogaia AB, 103 64 Stockholm (SE)
(72) Inventor: LUNDQVIST, Christoffer, S-226 49 Lund (SE)
(74) Representative: Allee, Harriet Eva Charlotta
(86) International application number: PCT/EP2010/059856
(87) International publication number: WO 2011/003995

(56) References cited:
- US-A1- 2003 006 159
- US-A1- 2007 160 789
- BASU SRIPARNA ET AL: "Efficacy of high-dose Lactobacillus rhamnosus GG in controlling acute watery diarrhea in Indian children: a randomized controlled trial", JOURNAL OF CLINICAL GASTROENTEROLOGY, RAVEN PRESS LTD., NEW YORK, NY, US, vol. 43, no. 3, 1 March 2009 (2009-03-01), pages 208-213, XP009140367, ISSN: 0192-0790
- GUANDALINI S ET AL: "Lactobacillus GG administered in oral rehydration solution to children with acute diarrhea: a multicenter European trial", JOURNAL OF PEDIATRIC GASTROENTEROLOGY AND NUTRITION, RAVEN PRESS, NEW YORK, NY, US, vol. 30, no. 1, 1 January 2000 (2000-01-01), pages 54-60, XP009140352, ISSN: 0277-2116
- SHORNIKOVA A V ET AL: "A TRIAL IN THE KARELIAN REPUBLIC OF ORAL REHYDRATION AND LACTOBACILLUS GG FOR TREATMENT OF ACUTE DIARRHOEA", ACTA PAEDIATRICA, UNIVERSITETSFORLAGET, OSLO, NO, vol. 86, 1 May 1997 (1997-05-01), pages 460-465, XP000199171, ISSN: 0803-5253

## Description

### FIELD OF THE INVENTION

The present invention relates generally to the packaging of products that are sensitive to moisture, consequently prolonging the shelf-life of such a product. More specifically this invention relates to prolonging the shelf-life of freeze dried lactic acid bacteria mixed with an oral rehydration solution (ORS) powder. The product is a package comprising of two desiccants: one desiccant integrated in the foil material and the second desiccant being anhydrous ORS. This invention also relates to methods for production of such products.

### BACKGROUND OF THE INVENTION

Probiotics are live microbial food supplements that beneficially affect the host by improving its intestinal microbial balance. Nowadays, a number of different bacteria are used as probiotics for example, lactic-acid producing bacteria such as strains of *Lactobacillus* and *Bifidobacteria.* Lactic-acid producing bacteria are not only used for their beneficial effect on human or animal health, but they are also widely used in the food industry for fermentation processes. Generally, the microorganisms marketed for these purposes are formulated as freeze-dried powders in a low water environment.

A general problem encountered in the application of such lyophilized microorganism preparations is the limited storage stability of the cells. For instance, over time the microorganisms become less viable resulting in high dosages being necessary to compensate for this loss of activity.

It is generally known to utilize aluminum foil as packaging material for the storage of microorganisms, and most preferably foil having a plastic layer on one surface on the outside of the compartment, such as a polyethylene laminated aluminum foil, as packaging material to reduce exposure of freeze dried lactic acid bacteria to moisture and oxygen.

Although the moisture barrier of conventional packages may be useful in restricting the movement of moisture into a package, some moisture molecules can still make their way into the package to deleteriously affect the product contained therein. In addition, even when barrier materials are effective at restricting the transmission of water molecules through a package, certain features of the package may still a low for the transmission of water molecules, for example, along the edges of a heat seated package. Also, the moment of packaging of the lyophilized microorganisms is a particularly weak point considering moisture absorption.

One solution to maintaining a particularly low or virtually nonexistent level of moisture within a package is to incorporate sachets of desiccant material into the internal space of the package to remove the moisture from the headspace of the package. The desiccant material contained in the sachets is typically in powder or granular form and may leak or otherwise spill from the sachets thereby contaminating the product or products contained within the package. Ingesting desiccants known in the market is not suitable for people suffering from diarrhea, for example. On the contrary it could create deleterious effects. However this issue is solved by the invention herein, wherein the purpose of the desiccant inside the sachet is for it to be consumed.

Typical desiccant materials are "physical" desiccant materials, such as molecular sieves, that bind water molecules within the pore spaces of a material. Typically, physical desiccant materials absorb water at all humidity levels, but will cease to absorb water when the interstices of the physical desiccant material are filled. Therefore, physical desiccant materials may be ineffective at high humidity levels.

Another type of desiccant material is hydrate-forming agents such as salts. Typical salts that may be utilized as desiccant material are magnesium surfate, sodium phosphate di-basic, ammonium chloride, potassium carbonate, potassium aluminum disulfate, magnesium chloride, diammonium sulfate, sodium nitrate, calcium chloride, and calcium sulfate, although many others are knomn as well. The drying capacity of these materials is greatly influenced by the relative humidity within a package. Generally, no water is taken up by the hydrate-forming agent until the relative humidity reaches a value at which the first hydrate forms. In the case of calcium chloride, for example, the first hydrate occurs at less than about two percent relative humidity (RH). Water is then taken up by the hydrate forming salt until the first hydrate is completely formed by the salt. No further water is taken up by the salt until the relative humidity reaches a second level where the second hydrate forms. This process continues through as many hydrates as the agent forms, at whith point the substance begins to dissolve and a saturated solution is formed. The saturated solution will then continue to take up water.

Although these salts may be effective at removing water molecules from a quantity of gas that may be contained within the headspace of a package, since the salt only binds the water molecules within the salt, the water molecules may easily escape back into the package. This is known as breathing, and may cause deliquescence (water droplets and liquidization) inside the package. Typically, this can happen if the salt becomes saturated and if the temperature of the package increases, or if the pressure of the package decreases, which may occur during shipment or storage of the package.

In addition, salts may not allow moisture levels within a package to fall to a level that is necessary to protect the moisture-sensitive product that may be contained within the package. Typically, since salts have different levels of hydration, humidity levels may remain at a certain level without decreasing until the level of hydration changes. These salts may be utilized to maintain certain humidity levels within the headspace of a package. For example, certain products may require that a certain level of moisture or humidity be maintained within the package headspace. Headspace humidity control for products can be manipulated by incorporation of the appropriate hydrate forming agents.

Desiccant materials may also be used that form no hydrates, such as common salt (NaCl) or potassium bromide (KBr). For example, common salt will absorb no water at a relative humidity below about 75 percent. When 75 percent relative humidity is reached, a saturated solution is formed which continues to take up water.

Another type of desiccant uses chemical desiccant technology. Chemical desiccant materials typically absorb water at all humidity levels, and will continue to take up water at high relative humidity levels. U.S. patent application No. 20070160789 A1 describes a multilayer plastic polymeric flexible packaging foil having the chemical desiccant material incorporated within a layer of the foil. The foil is preferably polyethylene selected from the group consisting of ultra low density polyethylene, low density polyethylene, linear low density polyethylene, medium density polyethylene, and high density polyethylene. This invention utilizes such desiccants as calcium oxide (preferred), magnesium oxide, barium oxide, strontium oxide, aluminum oxide, partially hydrated aluminum oxide, magnesium sulfate, sodium phosphate di-basic, ammonium chloride, potassium carbonate, potassium aluminum disulfate, magnesium chloride, diammonium sulfate, sodium nitrate, calcium chloride, calcium sulfate, sodium chloride, potassium bromide, molecular sieves, clays, or any other desiccant material useful for the present invention.

Oral rehydration therapy (ORT) is a simple, cheap, and effective treatment for dehydration associated with diarrhea. ORT consists of a solution of salts and sugars which are administered orally also called oral rehydration solution (ORS) and oral rehydration formula (ORF). ORT is used around the world, but is most important in the developing world, where it saves millions of children from death due to diarrhea-the second leading cause of death in children under five.

Several trials have been conducted to evaluate the efficacy lactic-acid bacteria administered in the oral rehydration solution. For example, a multicenter trial was conducted to evaluate the efficacy of *Lactobacillus* GG administered in the oral rehydration solution to patients with acute-onset diarrhea of all causes, showing that administering oral rehydration solution containing *Lactobacillus* GG to children with acute diarrhea is safe and results in shorter duration of diarrhea, less chance of a protracted course, and faster discharge from the hospital (J Pediatr Gastroenterol Nutr. 2000 Jan;30(1):54-60). However as mentioned before the problem is that freeze dried lactic acid bacteria require a low water activity (usually *a*_{w}<0.2) in a powder matrix to have a reasonable shelf life at ambient temperature without a dramatic loss in viability.

Due to the extremely hygroscopic (ability to attract water molecules from the surrounding environment) nature of oral rehydration solution powder, it has until now not been possible to mix a freeze dried lactic acid bacteria with an ORS powder and keep the bacteria viable during ambient storage for a longer period of time. The variable storage quality of live probiotic cultures results in unpredictable losses of dose and activity in the preparations administered to patients with acute watery diarrhea, and this is a technologic problem that is difficult to solve, particularly in developing countries.

From the storage point of view, a method was tested in the invention herein to evaluate whether any salt-mixtures could successfully work as desiccant inside a package, mixed with freeze dried *Lactobacillus reuteri*. The package was made of the foil material described in US20070160789 A1. Surprisingly the freeze-dried cultures of *L. reuteri* mixed with anhydrous ORS successfully reached at least 12 months of shelf life at 30°C which in known to mean longer storage times at lower temperatures. Advantageous storage stability data of this type have not been described hitherto. As far as we know there is no such product on the market anywhere in the world.

In summary, it has until now not been possible to mix a freeze dried lactic acid bacteria with an ORS powder and still keep the bacteria viable during ambient storage in any form of package for a longer period of time. The solution to this problem is solved by the invention herein by removing moisture in two steps with two different desiccants. The first desiccant is anhydrous ORS working as a desiccant, surrounding the microorganisms at the moment of packaging and the secondly desiccant is in the foil absorbing the moisture possibly arising during storage. When anhydrous ORS is used, it functions both as a desiccant that does not need to be removed simultaneously as it fulfills its aim as an oral rehydration solution.

### SUMMARY OF THE INVENTION

The invention herein provides a method of packaging of products that are sensitive to moisture, consequently prolonging the shelf-life of such a product, more specifically prolonging the shelf-life of freeze dried lactic acid bacteria mixed with oral rehydration solution (ORS) powder. The package comprises two desiccants: one desiccant integrated in the foil material and the second desiccant being anhydrous ORS.

A primary object of the present invention is to provide a product prolonging the shelf-lífe of freeze dried lactic acid bacteria mixed with oral rehydration solution (ORS) powder

Another object of the present invention is to provide a package with two desiccants removing moisture in two steps for the storage of freeze-dried lactic acid bacteria.

Another object is to provide a sachet with two desiccants: one desiccant integrated in the foil material, described in US20070160789 A1, and the other desiccant being anhydrous ORS, present freely inside the sachet, surrounding the product.

Other objects and advantages of the present invention will become obvious to the reader and it is intended that these objects and advantages are within the scope of the present invention.

### BRIEF DESCRIPTION OF THE DRAWINGS

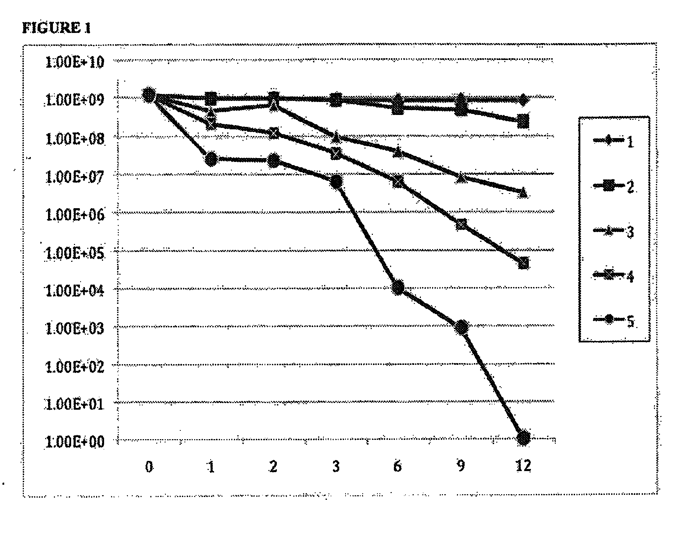

Figure 1 is a graph showing the viability of *Lactobacillus reuteri* with different desiccants at +30°C on a monthly basis up to 12 months:
   1) *L.reuteri* + foil with desiccant
   2) *L.reuteri* + foil with desiccant + anhydrous ORS
   3) *L.reuteri* + foil with desiccant + non-anhydrous ORS
   4) *L.reuteri* + foil without desiccant + anhydrous ORS
   5) *L.reuteri* + foil without desiccant + non-anhydrous ORS

### DETAILED DESCRIPTION OF THE INVENTION AND PREFERRED EMBODIMENTS THEREOF

The method and device of the invention provide packaging of products that are sensitive to moisture, consequently prolonging the shelf-life of such a product, more specifically prolonging the shelf-life of freeze dried lactic acid bacteria mixed with oral rehydration solution (ORS) powder. The package comprises two desiccants: one desiccant integrated in a aluminum foil and the second desiccant being anhydrous ORS. The foil is preferably made of aluminum, with a suitable protective coating such as polyethylene or a lacquer. The reason being is that aluminum foil is by far the best known barrier against moisture and the products discussed herein are very sensitive to moisture. Testing of the barrier charactcristics of the package is done as known in the art (Allinson et al, International Journal of Pharmaceutics Volume 221, Issues 1-2, 19 June 2001, Pages 49-5)

The term "anhydrous ORS" as used herein means that the components of the ORS powder are of anhydrous quality i.e. free of water. The components can be bought in anhydrous form or can be made anhydrous by methods known in the art.

The goal of the invention herein is to provide a method and device that results in survival of the freeze dried lactic acid bacteria such that at the end of 12 months, at storage around +30°C there are at least 10E+07 CFU/gram (when starting with 5x10E+09CFU/gram) but preferably at least 10E+08 CFU/gram and best 10E+09 CFU/gram at this temperature and starting point. This stability is known to translate to maximum the same amount of losses at around 18 months when the product is stored in +25 °C and less when the temperature is lower. This would mean that the goal at 18 month at +25°C there are at least 10E+07 CFU/gram (when starting with 5x10E+09CFU/gram) but preferably at least 10E+08 CFU/gram and best 10E+09 CFU/gram at this temperature and starting point.

The features of the present invention will be more clearly understood by reference to the following examples, which are not to be construed as limiting the invention.

### EXAMPLE 1

### Parameters tested

In the examples herein, the method of the invention is tested to evaluate if ORS solution salts can be mixed with freeze dried *L. reuteri* in a sachet with the aim of reaching at least 12 months of shelf life at 30°C as an accelerated study which in known to mean longer storage times at lower temperatures. The new production method includes the use of a new foil material, with a desiccant, described in US20070160789 A1.

The assays undertaken consist of five different test parameters as shown in Table 1:

**Table 1**

| Test parameter | *L. reuteri* DSM 17938 | ORS anhydrous: | ORS | PET12/PE/ALU 12/PE/PE+desiccant/PE from Alcan * | PETP 12/ALU 9/LLDPE70 from Amcor Flexibles ** |
|---|---|---|---|---|---|
| 1 | X | | | X | |
| 2 | X | X | | X | |
| 3 | X | | X | X | |
| 4 | X | X | | | X |
| 5 | X | | X | | X |

| | | | | | |
|---|---|---|---|---|---|
| * Foil with integrated desiccant desribed in **US20070160789 A1** ** Reference foil without integrated desiccant | | | | | |

### EXAMPLE 2

### Production of sachets comprising a mik of L. reuteri in anhydrous O.R.S powder

The anhydrous ORS powder contained:
*L.reuteri* DSM 17938:68.000 mg/sachet at around 10E+11 CFU/gram
Glucose anhydrous: 3781.000 mg/sachet
Sodium citrate anhydrous: 866.000 mg/sachet
Potassium chloride: 379.000 mg/sachet
Sodium chloride: 365.000 mg/sachet
Zinc sulphate: 4.000 mg/sachet
TOTAL 5463.000 mg/sachet

The blend was filled at ambient temperature into aluminum foil bags with desiccant (10 cm x 12 cm, using packaging material PET12/PE/ALU 12/PE/PE+desiccant/PE from Alcan) in a LAF bench (Holten Laminair Model S-2010 1.2 from Heto-Holten A/S, Denmark) at the microbiological laboratory at BioGaia Lund. To each bag 5.46 g of ORS powder with *L*. *reuteri* was added using the balance XP-600 from Denser Instrument GmbH, Germany. The filled aluminum foil bags were then heat sealed with the foil sealing device model F460/2 from Kettenbaum Folienschweisstechnik GmbH & Co. KG, Germany. As there is practically no permeability of moisture at all trough the alunimum foil, especially if the alumimum is thicker than 0.025 mm and therefore most prefered, the seal is the sensitive part of the total enclosure, which means that the sealing device use must be of high quality giving unbroken seals without any leaks and sufficient with as known in the art. These bags were also used in the study described in example 3.

### EXAMPLE 3

### Stability study of freeze dried L. reuteri DSM 17938 during storage in oral rehydration solution (ORS) powder at 30°C, during 12 months.

The viability of *L. reuteri* DSM 17938 powder art no 1012 BioGaia AB, Stockholm, Sweden (product specification PS137) is evaluated in an oral rehydration solution powder from Norfoods AB, Sweden. The anhydrous ORS powder is made according to example 2. The test parameters are performed according to table 1. The samples are stored in climate cabinets at 30°C/65% RH at BioGaia ABn in Lund, Sweden.

### EXAMPLE 4

The results are shown in figure 1. The microorganisms stored in the foil with integrated desiccant and anhydrous ORS suprisingly (graph 2 in figure 1) showed almost the same viability as the microorganisms stored without ORS (graph 1 in figure 1).

The microorganisms stored in the foil with integrated desiccant showed better viability during storage than the microorganisms stored in the foil without desiccant, since the desiccant in the foil absorbs almost all of the moisture within the bag during storage. But only having the foil with desiccant and non-anhydrous ORS (graph 3 in figure 1) is shown to not be enough since the viability is poorer than with the microorganisms stores in the foil with integrated desiccant and anhydrous ORS (graph 2 in figure 1)

Conclusively, neither the anhydrous ORS alone nor the foil with integrated desiccant alone is enough to provide a product with sufficient shelf-life. Storage including both of them together fulfills the activity goal at the end of shelf life.

## Claims

1. A probiotic product, comprising: freeze-dried lactic acid bacteria mixed with anhydrous oral rehydration solution powder, the freeze-dried lactic acid bacteria and the anhydrous oral rehydration solution powder packaged in a foil having desiccant integrated in the foil.

2. The probiotic product of claim 1, wherein the foil comprises aluminum.

3. The probiotic product of claim 2, wherein the foil further comprises a polyethylene layer.

4. The probiotic product of claim 1, wherein the desiccant integrated in the foil material comprises calcium oxide.

5. The probiotic product of claim 1, wherein the freeze-dried lactic acid bacteria are *Lactobacillus reuteri.*

6. The probiotic product of claim 1, wherein after 12 months of storage of the probiotic product at 30°C there are at least 10E+07 CFU/gram of *Lactobacillus reuteri,* when starting with 5x10E+09CFU/gram of *Lactobacillus reuteri.*

7. A product for the viable storage of a product that is sensitive to moisture, comprising:
anhydrous oral rehydration solution powder for mixing with the product that is sensitive to moisture, and packaging for the product that is sensitive to moisture and anhydrous oral rehydration solution powder comprising a chemical desiccant material incorporated within a layer of foil.

8. The product of claim 7, wherein the product that is sensitive to moisture comprises viable freeze-dried lactic acid bacteria.

9. The product of claim 7, wherein the foil comprises aluminum.

10. The product of claim 9, wherein the foil further comprises a polyethylene layer.

11. A method of long-term storage of viable freeze-dried probiotic lactic acid bacteria, comprising:
a) providing anhydrous oral rehydration solution powder;
b) mixing freeze-dried lactic acid bacteria with the anhydrous oral rehydration solution powder;
c) packaging the mixed freeze-dried lactic acid bacteria and oral rehydration powder in a package comprising a chemical desiccant material incorporated within a layer of foil; and
d) sealing the packages so that the seal is unbroken.

12. The method of claim 11, wherein the foil comprises aluminum foil.

13. The method of claim 12, wherein the foil further comprises a polyethylene layer.

14. The method of claim 11, wherein the desiccant integrated in the foil material comprises calcium oxide.

15. The method of claim 11, wherein the freeze-dried lactic acid bacteria are *Lactobacillus reuteri.*

## Patentansprüche

1. Probiotisches Produkt, umfassend: gefriergetrocknete Milchsäurebakterien gemischt mit wasserfreiem Pulver einer oralen Rehydrationslösung, wobei die gefriergetrockneten Milchsäurebakterien und das wasserfreie Pulver einer oralen Rehydrationslösung in einer Folie verpackt sind, wobei in die Folie ein Trocknungsmittel integriert ist.

2. Probiotisches Produkt nach Anspruch 1, wobei die Folie Aluminium umfasst.

3. Probiotisches Produkt nach Anspruch 2, wobei die Folie weiterhin eine Polyethylenschicht umfasst.

4. Probiotisches Produkt nach Anspruch 1, wobei das Trocknungsmittel, das in das Folienmaterial integriert ist, Calciumoxid umfasst.

5. Probiotisches Produkt nach Anspruch 1, wobei die gefriergetrockneten Milchsäurebakterien *Lactobacillus reuteri* sind.

6. Probiotisches Produkt nach Anspruch 1, wobei nach 12 Monaten Lagerung des probiotischen Produkts bei 30°C mindestens 10E+07 CFU/Gramm *Lactobacillus reuteri* vorhanden sind, wenn von 5x10E+09 CFU/Gramm *Lactobacillus reuteri* ausgegangen wird.

7. Produkt für die lebensfähige Lagerung eines Produkts, das sensitiv gegenüber Feuchtigkeit ist, umfassend: wasserfreies Pulver einer oralen Rehydrationslösung zur Mischung mit dem Produkt, das sensitiv gegenüber Feuchtigkeit ist, und eine Verpackung für das Produkt, das sensitiv gegenüber Feuchtigkeit ist und für das wasserfreie Pulver einer oralen Rehydrationslösung, umfassend ein chemisches Trocknungsmittel, das in eine Folienschicht integriert ist.

8. Produkt nach Anspruch 7, wobei das Produkt, das sensitiv gegenüber Feuchtigkeit ist, lebensfähige, gefriergetrocknete Milchsäurebakterien umfasst.

9. Produkt nach Anspruch 7, wobei die Folie Aluminium umfasst.

10. Produkt nach Anspruch 9, wobei die Folie weiterhin eine Polyethylenschicht umfasst.

11. Verfahren zur Langzeitlagerung von probiotischen, lebensfähigen, gefriergetrockneten Milchsäurebakterien, umfassend:
a) Bereitstellen eines wasserfreien Pulvers einer oralen Rehydrationslösung;
b) Mischen von gefriergetrockneten Milchsäurebakterien mit wasserfreiem Pulver einer oralen Rehydrationslösung;
c) Verpacken der gefriergetrockneten Milchsäurebakterien, die mit dem wasserfreien Pulver einer oralen Rehydrationslösung gemischt sind, in einer Verpackung, umfassend ein chemisches Trocknungsmittel, das in eine Folienschicht integriert ist; und
d) Versiegeln der Verpackungen, so dass das Siegel unversehrt ist.

12. Verfahren nach Anspruch 11, wobei die Folie Aluminiumfolie umfasst.

13. Verfahren nach Anspruch 12, wobei die Folie weiterhin eine Polyethylenschicht umfasst.

14. Verfahren nach Anspruch 11, wobei das Trocknungsmittel, das in das Folienmaterial integriert ist, Calciumoxid umfasst.

15. Verfahren nach Anspruch 11, wobei die gefriergetrockneten Milchsäurebakterien *Lactobacillus reuteri* sind.

## Revendications

1. Produit probiotique, comprenant : des bactéries lactiques lyophilisées mélangées avec une poudre de solution de réhydratation orale anhydre, les bactéries lactiques lyophilisées et la poudre de solution de réhydratation orale anhydre étant emballées dans une feuille ayant un dessiccatif intégré dans la feuille.

2. Produit probiotique selon la revendication 1, dans lequel la feuille comprend de l'aluminium.

3. Produit probiotique selon la revendication 2, dans lequel la feuille comprend en outre une couche de polyéthylène.

4. Produit probiotique selon la revendication 1, dans lequel le dessiccatif intégré dans le matériau de la feuille comprend de l'oxyde de calcium.

5. Produit probiotique selon la revendication 1, dans lequel les bactéries lactiques lyophilisées sont des *Lactobacillus reuteri.*

6. Produit probiotique selon la revendication 1, dans lequel après 12 mois de stockage du produit probiotique à 30°C, on a au moins 10E+07 CFU/gramme de *Lactobacillus reuteri,* en démarrant avec 5 x 10E+09 CFU/gramme de *Lactobacillus reuteri.*

7. Produit destiné au stockage viable d'un produit qui est sensible à l'humidité, comprenant :
une poudre de solution de réhydratation orale anhydre destinée à se mélanger avec le produit qui est sensible à l'humidité, et un emballage pour le produit qui est sensible à l'humidité et la poudre de solution de réhydratation orale anhydre comprenant un matériau dessiccatif chimique incorporé au sein d'une couche de feuille.

8. Produit selon la revendication 7, dans lequel le produit qui est sensible à l'humidité comprend des bactéries lactiques lyophilisées viables.

9. Produit selon la revendication 7, dans lequel la feuille comprend de l'aluminium.

10. Produit selon la revendication 9, dans lequel la feuille comprend en outre une couche de polyéthylène.

11. Procédé de stockage à long terme de bactéries lactiques probiotiques lyophilisées viables, comprenant les étapes consistant à :
a) fournir une poudre de solution de réhydratation orale anhydre ;
b) mélanger des bactéries lactiques lyophilisées avec la poudre de solution de réhydratation orale anhydre ;
c) emballer les bactéries lactiques lyophilisées et la poudre de réhydratation orale mélangées dans un emballage comprenant un matériau dessiccatif chimique incorporé au sein d'une couche de feuille ; et
d) sceller les emballages de sorte que le sceau soit non brisé.

12. Procédé selon la revendication 11, dans lequel la feuille comprend du papier d'aluminium.

13. Procédé selon la revendication 12, dans lequel la feuille comprend en outre une couche de polyéthylène.

14. Procédé selon la revendication 11, dans lequel le dessiccatif intégré dans le matériau de la feuille comprend de l'oxyde de calcium.

15. Procédé selon la revendication 11, dans lequel les bactéries lactiques lyophilisées sont des *Lactobacillus reuteri.*
